# FASCICULE DE BREVET EUROPEEN

(11) **EP 3 442 684 B1**
(45) Date de publication et mention de la délivrance du brevet: **13.08.2025**
(21) Numéro de dépôt: 17722101.7
(22) Date de dépôt: 13.04.2017
(51) Int. Cl.: B01D 53/52

(54) **NOUVEAU PROCÉDÉ D'ÉPURATION OU DE DÉSODORISATION DE GAZ**
NEUARTIGES VERFAHREN ZUR REINIGUNG ODER DESODORIERUNG VON GAS
NOVEL METHOD FOR PURIFYING OR DEODORIZING GAS

(30) Priorité: 14.04.2016 FR 1653284
(43) Date de publication de la demande: 20.02.2019
(73) Titulaire: Deltalys, 69190 Saint Fons (FR); Institut National des Sciences Appliquées de Lyon, 69621 Villeurbanne Cedex (69266) (FR)
(72) Inventeur: GERMAIN, Charly, 69007 Lyon (FR); GERMAIN, Patrick, 69001 Lyon (FR); BENBELKACEM, Hassen, 69004 Lyon (FR)
(74) Mandataire: Germain Maureau
(86) Numéro de dépôt international: PCT/FR2017/050892
(87) Numéro de publication internationale: WO 2017/178766

(56) Documents cités:
- WO-A1-2007/077924
- WO-A2-2009/039393
- CN-A- 1 775 344
- CN-B- 102 114 379
- JP-A- H09 215 736
- KR-A- 20120 033 073
- US-A- 2 204 113

## Description

La présente invention a pour objet un nouveau procédé d'épuration ou de désodorisation de gaz par mise en contact avec une matrice réactive spécifique.

Le biogaz est le gaz produit par la fermentation de matières organiques animales ou végétales en l'absence d'oxygène. Cette fermentation appelée aussi méthanisation se produit naturellement (dans les marais) ou spontanément dans les décharges contenant des déchets organiques, mais on peut aussi la provoquer artificiellement dans des digesteurs (pour traiter des boues d'épuration, des déchets organiques industriels ou agricoles, etc...).

Le biogaz est principalement composé de méthane, de gaz carbonique et d'autres gaz à l'état de traces, en particulier d'hydrogène sulfuré (H₂S) et de mercaptans ainsi que de composés volatils du silicium comme des siloxanes.

Quelle que soit son origine, le biogaz non valorisé contribue, du fait de ses fortes teneurs en méthane, à l'effet de serre. De plus, le biogaz non valorisé est généralement considéré comme une source de nuisances, notamment en raison :
- de risques pour la santé humaine liés à la présence de gaz à l'état de trace, appartenant, entre autres, à des familles chimiques tels que les BTEX (Benzène, Toluène, Ethylène, et Xylène), les composés organiques chlorés et fluorés, les composés contenant du soufre ou les composés de la famille des terpènes ;
- des odeurs nauséabondes qui accompagnent son dégagement. En effet, même l'état de traces (ppm voire ppb), des composés soufrés (hydrogène sulfuré, polysulfure, etc.), des acides et des aldéhydes, présents dans le biogaz et véhiculés par lui, ainsi que parfois du xylène, du toluène, du chlorure de vinyle, etc., sont responsables d'odeurs désagréables ;
- des risques d'explosion, dans la mesure où 5 à 15% de méthane dans l'air peuvent constituer dans certaines conditions un mélange explosif.

Cependant, le biogaz permet également la production d'énergie. L'énergie du biogaz provient principalement du méthane : le biogaz est ainsi la forme renouvelable de l'énergie fossile très courante qu'est le gaz naturel qui, lui, contient essentiellement du méthane mais aussi du butane, du propane et d'autres éléments.

La présence d'hydrogène sulfuré, mercaptans et composés siliciés dans le biogaz constitue donc une source de nuisance pour les procédés de valorisation ou de combustion du biogaz qui doit être épuré de ses composants nuisibles, en particulier l'hydrogène sulfuré et les mercaptans, les composés siliciés comme les siloxanes et éventuellement le gaz carbonique. Cette étape d'épuration permet d'amener le biogaz à une qualité suffisante pour permettre sa valorisation, que celle-ci soit directe dans un organe de combustion, ou indirecte via la production de biométhane que l'on peut injecter dans le réseau de distribution ou de transport du gaz naturel.

Dans l'optique d'obtention de biométhane, un procédé supplémentaire sophistiqué de raffinage du biogaz en biométhane est nécessaire et reste assez coûteux mais présente une grande marge de progrès. Ce gaz est malgré tout une des sources renouvelables d'énergie intéressant pour la transition énergétique.

Quel que soit le mode de valorisation préférentiel du gaz choisi, différentes techniques d'épuration peuvent être utilisées. L'adsorption physique ou réactive mettant en œuvre des matériaux solides - généralement poreux - est couramment utilisée industriellement. Toutefois, il s'agit d'une opération onéreuse dans la chaîne de valorisation énergétique, notamment à cause du coût des matériaux commerciaux et du manque d'optimisation des installations.

D'autres procédés de traitement des biogaz ont ainsi été décrits récemment.

La demande de brevet français FR-A-2982175 décrit une installation permettant la mise en œuvre d'un procédé d'épuration de biogaz par mise en contact dudit biogaz avec du mâchefer d'incinération de déchets non dangereux.

La demande de brevet FR-A-3019061 décrit quant à elle un procédé de purification de biogaz par mise en contact dudit biogaz enrichi en air ou en oxygène dans au moins un adsorbeur comprenant du charbon actif imprégné sur lequel s'adsorbe préférentiellement l'hydrogène sulfuré.

Enfin, la demande de brevet EP-A-2457982 décrit un procédé de traitement de biogaz par mise en contact dudit biogaz avec des cendres volantes.

Cependant, aucun des procédés décrits dans ces demandes de brevet ne donne entière satisfaction en termes d'efficacité. En effet, ces procédés sont très onéreux à mettre en œuvre en comparaison de leur efficacité ce qui est pénalisant pour l'économie de la filière. D'autre part, ces procédés sont standardisés et ne peuvent de ce fait répondre correctement à l'épuration efficace de biogaz ou biométhane d'origines et de compositions moléculaires dont la variabilité n'est pas prise en compte

En outre, les problématiques rencontrées avec les biogaz se retrouvent également lorsque l'on a affaire à des gaz naturels, des gaz de synthèse, du biométhane ou, plus généralement, tout autre gaz contenant des composés tels que le sulfure d'hydrogène, des mercaptans, des composés siliciés tels que les siloxanes, des composés organiques volatils (COV), du dioxyde de carbone ou tout autre composé minoritaire indésirable responsables de problèmes d'odeurs, de pollution ou de dangerosité.

A la date de la présente invention, il demeure donc nécessaire d'identifier des procédés et des réactifs permettant d'améliorer l'efficacité des traitements des biogaz, et plus généralement des gaz, en vue de leur dépollution et/ou de leur désodorisation.

Pour répondre à ces difficultés particulières, certains procédés de traitement mettant en œuvre une matrice contenant du SiO₂ et du CaO dans des teneurs variables ont été décrits.

Ainsi, la demande de brevet chinois CN 1775344 décrit un procédé d'épuration et de désodorisation de gaz, notamment de désulfurisation, par mise en contact dudit gaz à traiter avec une matrice comprenant 10% en poids de SiO2, 6% à 15% en poids de CaO 7% à 12% en poids d'eau. Le procédé décrit dans cette demande de brevet est un procédé de traitement « à sec », c'est-à-dire mettant en œuvre une matrice contenant peu ou pas d'eau.

La demande de brevet coréen KR 20120033073 décrit quant à elle un procédé de désulfurisation d'un gaz par mise en contact dudit gaz avec une matrice comprenant de 1% à 3% en poids de SiO₂, de 58% à 65% en poids de CaO et 28% à 39% en poids d'eau et d'impuretés. Selon cette demande de brevet, « lorsque le taux de CaO est inférieur à 58%, l'efficacité de la désulfuration est extrêmement faible, et lorsque le taux de CaO est supérieur à 65%, le gain d'efficacité n'est pas proportionnelle à l'augmentation du taux de CaO, ce qui ne présente donc pas d'intérêt ».

Enfin, la demande de brevet américain US 2,204,113 suggère différentes utilisations industrielles d'un silicate de calcium hydraté comprenant 76% en poids de matière sèche de SiO₂, 24% en poids de matière sèche de CaO, et dont le taux d'humidité est de 18,5%, parmi lesquelles l'utilisation comme pigment, comme agent activant, comme agent décolorant ou comme absorbant sélectif pour le traitement de gaz. Cependant, ce document ne fournit aucune explication quant à la façon dont une telle utilisation pourrait être mise en œuvre.

Or, il a maintenant été trouvé, de façon tout à fait surprenante, que l'utilisation d'une matrice humide contenant moins de 50% de CaO dans un procédé de traitement des gaz permettait de réduire plus efficacement la teneur des composants nuisibles qu'ils contiennent (voire de les éliminer totalement), en particulier le sulfure d'hydrogène, les mercaptans, les composés siliciés tels que les siloxanes, les composés organiques volatils (COV), les composés chlorés ou fluorés et le dioxyde de carbone.

La présente invention a donc pour objet un procédé d'épuration ou de désodorisation de gaz comprenant la mise en contact dudit gaz avec une matrice réactive comprenant :
- de 15% à 80% en poids de matière sèche de SiO₂; et
- de 1% à 40% en poids de matière sèche de CaO ;
le taux d'humidité de ladite matrice variant de 20% à 80%.

Le procédé selon la présente invention permet de réduire significativement la teneur voire d'éliminer les composants nuisibles qu'ils contiennent, en particulier le sulfure d'hydrogène, les mercaptans, les composés siliciés tels que les siloxanes, les composés organiques volatils (COV), les composés chlorés ou fluorés et le dioxyde de carbone. En outre, le procédé selon la présente invention présente également l'avantage de permettre une régénération (totale ou partielle) de la matrice par simple mise en contact avec l'air.

Dans le cadre de la présente invention :
- on entend par « gaz » tout gaz ou flux gazeux contenant du sulfure d'hydrogène, des mercaptans, des composés siliciés tels que les siloxanes, des composés organiques volatils (COV), des composés chlorés ou fluorés ou du dioxyde de carbone, en particulier le gaz naturel, le gaz de synthèse, le biogaz, le biométhane, l'air pollué et les fumées de combustion ;
- on entend par « gaz naturel » tout gaz combustible fossile constitué d'un mélange d'hydrocarbures dont le méthane (CH₄) est l'un des principaux composants ;
- on entend par « gaz de synthèse » ou « syngaz » tout mélange gazeux combustible produit par pyrolyse de matière organique ou partiellement organique (charbon, biomasse végétale, biodéchets). Les constituants principaux de ces gaz sont le monoxyde de carbone (CO) et l'hydrogène (H₂) ;
- on entend par « biogaz » tout gaz produit par fermentation anaérobie de matière organique (biomasse végétale, biodéchets) dans un méthaniseur, un digesteur ou une installation de stockage. Le biogaz est constitué majoritairement de méthane (CH₄) et de dioxyde de carbone (CO₂) ;
- on entend par « biométhane » tout gaz, aux standards du gaz naturel (possédant notamment un pouvoir calorifique inférieur -PCI- au moins égal à 10 KWh/Nm³), issu de l'épuration et de l'enrichissement d'un biogaz, ou d'un syngaz transformé par une étape dite de méthanation ;
- on entend par « fumée » tout flux gazeux qui se dégage de tout corps ou gaz en combustion, cette combustion pouvant être totale ou partielle ;
- on entend par « air pollué » tout air contenant du sulfure d'hydrogène, des mercaptans, des composés siliciés tels que les siloxanes, des composés organiques volatils (COV) ou des composés chlorés ou fluorés consécutivement à une mise en contact ou mélange avec un gaz ou produit solide pouvant contenir ces mêmes composés ;
- on entend par « procédé d'épuration ou de désodorisation d'un gaz » tout procédé permettant de diminuer d'au moins 15%, de préférence au moins 20%, la quantité de sulfure d'hydrogène, de mercaptans, de composés siliciés, de composés organiques volatils (COV), de composés chlorés ou fluorés et/ou de dioxyde de carbone que ledit gaz contient ;
- on entend par « composés siliciés » tout composé organique du silicium tel que les siloxanes, les silanes ou les silarènes De préférence, on entend par « composés siliciés » les siloxanes.
- on entend par « composés organiques volatils » (ou COV) tout composé tel que défini à l'article 2 de la Directive 1999/13/CE, à savoir tout composé organique ayant une pression de vapeur de 0,01 kPa ou plus à une température de 293,15 K ou ayant une volatilité correspondante dans les conditions d'utilisation particulières, en particulier les composés aromatiques (dérivés du benzène), les composés organochlorés , les composés organofluorés, HCN, HCl, l'ammoniac, les alcanes, les alcènes, les terpènes et les composés oxygénés ;
- on entend par « matrice réactive » toute matrice composée de sous-produits ou résidus issus de procédés industriels, mélangés ou non, sous quelque forme que ce soit, et dont la fonction est de piéger tout ou partie des composés néfastes présents dans le gaz. Le piégeage de ces composés peut être dû à une réaction chimique (chimisorption) ou physique (physisorption) ;
- on entend par « pH de la matrice réactive » le pH du liquide résultant de la mise en contact de la matrice solide avec de l'eau pure dans un rapport massique liquide/solide égal à 10 ;
- on entend par « matière sèche » toute matière brute composée de matière minérale et organique séchée à 105°C jusqu'à poids constant;

Dans le cadre de la présente invention, le « % en poids de matière sèche » ou « %MS » d'un constituant par rapport à une composition (e.g. une matrice) désigne le rapport du poids dudit constituant sur le poids de matière sèche totale de ladite composition.

Dans le cadre de la présente invention le « taux de CaO en poids matière sèche » désigne le rapport du poids de calcium exprimé en oxyde sur le poids de matière sèche totale.

Dans le cadre de la présente invention, le « taux d'humidité » d'une composition (e.g. une matrice) désigne le rapport du poids de l'eau, à l'exception de l'eau de cristallisation, contenue dans la composition sur le poids total de ladite composition.

Dans le cadre de la présente invention, on entend par « eau de cristallisation » l'eau spécifiquement liée à certains composés de la matrice et ne pouvant pas jouer le rôle de solvant. Les composés possédant ces molécules d'eau intégrées dans leur structure cristalline sont dénommés « hydrates définis ».

Dans le cadre de la présente invention, « l'humidité relative » d'un gaz désigne le rapport entre la pression partielle de vapeur d'eau dans ce gaz et la pression maximale de vapeur d'eau qu'il peut contenir dans des conditions de température et de pression déterminées. A titre d'exemple, si l'humidité relative d'un gaz est de 50%, à une température et pression donnée, ledit gaz ne contient que la moitié de la quantité maximale de vapeur d'eau qu'il peut contenir dans ces conditions.

Dans le cadre de la présente invention, le « point de rosée » ou « température de rosée » d'un gaz désigne la température à laquelle l'humidité relative de ce gaz devient égale à 100%. Il s'agit donc de la température à laquelle la pression partielle de vapeur d'eau dans un gaz est égale à la pression maximale de vapeur d'eau qu'il peut contenir.

Dans le cadre de la présente invention, l'expression « poudre (ou mélange de poudres) dont la granulométrie est comprise entre 'x' µm et 'y' µm » désigne tout additif sous forme de poudre dont au moins 50% (en volume) des particules ont un diamètre compris entre 'x' µm et 'y' µm, le diamètre des particules pouvant être déterminés par toute méthode connue de l'homme du métier, notamment par tamisage.

Dans le cadre de la présente invention, l'expression « granulés (ou « pellets ») dont la taille est comprise entre 'x' µm et 'y' µm » désigne tout additif sous forme de granulés dont la longueur est comprise entre 'x' µm et 'y' µm, la longueur des granulés pouvant être déterminée par toute méthode connue de l'homme du métier, notamment par tamisage et analyse granulométrique.

Le procédé selon la présente invention comprend donc une étape de mise en contact du gaz à traiter avec une matrice réactive telle que définie précédemment. De préférence, la matrice réactive utilisée dans le cadre du procédé selon la présente invention présente les caractéristiques suivantes, prises seules ou en combinaison :
- la matrice réactive contient de 15%MS à 60%MS de SiO₂, de préférence encore de 18%MS à 40%MS de SiO₂;
- la matrice réactive contient de 10%MS à 40%MS de CaO, de préférence encore de 20%MS à 40%MS de CaO ;
- le taux d'humidité de la matrice réactive varie de 20% à 60% ;
- le pH de la matrice réactive est supérieur ou égal à 7, de préférence encore supérieur ou égal à 10, de façon tout à fait préférée supérieur ou égal à 12 ;
- la matrice réactive se présente sous la forme d'une poudre ou d'un mélange de poudres dont la granulométrie varie de 0,1 µm à 15 cm ; de préférence encore de 0,5 µm à 1000 µm ;
- la matrice réactive se présente sous la forme de granulés dont la taille varie de 0,1 cm à 25 cm ; de préférence encore de 0,5 cm à 5 cm; et/ou
- la matrice réactive comprend en outre un ou plusieurs des constituants suivants :
   > jusqu'à 60%MS de Fe₂O₃ ; de préférence encore de 10%MS à 50%MS de Fe₂O₃ ;
   ➢ jusqu'à 30%MS d'Al₂O₃ ; de préférence encore de 4%MS à 20%MS de Al₂O₃ ;
   ➢ jusqu'à 35%MS de Na₂O ; de préférence encore de 1%MS à 10%MS de Na₂O ;
   ➢ jusqu'à 50%MS de K₂O ; de préférence encore de 2%MS à 15%MS de K₂O ;
   ➢ jusqu'à 15%MS de TiO₂ ; de préférence encore de 1%MS à 10%MS de TiO₂ ;
   ➢ jusqu'à 60%MS de MgO; de préférence encore de 3%MS à 9%MS de MgO ;
   ➢ jusqu'à 30 %MS de SO₃ ; de préférence encore de 1%MS à 6%MS de SO₃ ;
   ➢ jusqu'à 15%MS de P₂O₅ ; de préférence encore de 1%MS à 6%MS de P₂O₅ ; et/ou
   > jusqu'à 60%MS de C.

En plus des constituants cités précédemment, la matrice réactive utilisée dans le procédé selon la présente invention peut également comprendre un ou plusieurs des composés suivants :
➢ jusqu'à 10%MS d'As ;
➢ jusqu'à 10%MS de Sb ;
➢ jusqu'à 10%MS de Cd;
➢ jusqu'à 10%MS de Cr;
➢ jusqu'à 10%MS de Co;
➢ jusqu'à 10%MS de Cu;
➢ jusqu'à 10%MS de Mn ;
➢ jusqu'à 10%MS de Ni;
➢ jusqu'à 10%MS de Pb;
➢ jusqu'à 10%MS de V;
➢ jusqu'à 10%MS de Zn ;
➢ jusqu'à 10%MS de F ; et/ou
➢ jusqu'à 10%MS de Cl.

La matrice réactive utilisée dans le procédé selon la présente invention peut être préparée par mélange homogène ou hétérogène de matériaux ou strates distinctes choisis parmi des déchets non recyclables en fin de vie (orphelins de filière), choisis pour leur composition et les propriétés physico chimiques de leurs composés tels que les poudrettes de pneus usagés, les laitiers d'aciérie, les boues rouges, les boues métalliques, les déchets de tannerie, les boues de STEP, les cendres de biomasse, les résidus (cendres) de gazéification.

De façon préférée, la matrice réactive utilisée dans le procédé selon la présente invention est obtenue à partir de matériaux choisis parmi :
- les cendres de biomasse produites à partir de la combustion de biomasse de type ligno-cellulosique (bois de classe A ou B) dans les unités de type "chaufferie bois" : cendres sous foyer et/ou cendres volantes;
- les cendres de gazéification, produites à partir de la pyrolyse de matériaux organiques tels que le bois de classe A ou B ou les déchets ménagers : cendres volantes et/ou cendres sous foyer ; et/ou
- les boues métalliques (ou boues rouges), résidus de procédés de fabrication de l'alumine. Ces boues peuvent être préalablement déshydratées par le producteur ;
- les pouzzolanes ou matériaux équivalents de type déchets pouzzolaniques.

La granulométrie de la matrice réactive utilisée dans le procédé selon la présente invention est susceptible de varier de manière importante, celle-ci étant en effet susceptible de se présenter sous forme de poudre plus ou moins fine ou de granulés (ou pellets) de taille supérieure.

Le procédé selon la présente invention comprend donc une étape de mise en contact du gaz à traiter avec une matrice réactive telle que définie précédemment. De préférence, le procédé selon la présente invention est conduit dans les conditions suivantes, prises seules ou en combinaison :
- le gaz à traiter possède une humidité relative avant mise en contact avec la matrice réactive supérieure ou égale à 60%, de préférence supérieure ou égale à 80%, et un point de rosée dans les conditions de pression du gaz supérieur ou égal à 4°C, de préférence supérieur ou égal à 15°C. Au besoin, le gaz pourra être humidifié préalablement à sa mise en contact avec la matrice, par exemple être mis en contact avec de l'eau brute jusqu'à obtention du taux d'humidité et du point de rosée souhaité ;
- le gaz à traiter est mis en contact avec la matrice réactive pendant une durée supérieure à 3 secondes, de préférence supérieure à 10 secondes, de préférence encore supérieure à 20 secondes ;
- la pression absolue du gaz à traiter est comprise entre 100 millibars et 400 bars, de préférence entre 800 millibars et 10 bars ;
- le gaz à traiter est préalablement humidifié par mise en contact avec de l'eau brute, qui peut être liquide ou pulvérisée sous forme de brouillard.

Le procédé selon la présente invention peut être mis en œuvre à l'aide de toute installation classiquement utilisée et connue de l'homme du métier pour l'épuration ou la désodorisation de gaz.

En fonction des gaz à traiter et du type d'épuration ou de désodorisation envisagée, la matrice réactive pourra par exemple être placée dans différents réacteurs ou contenants, ayant pour propriétés communes la possibilité de faire circuler un flux de gaz à travers la matrice et de retenir sa charge polluante ou indésirable. A titre d'exemples de tels réacteurs ou contenants, on peut notamment citer des silos verticaux ou horizontaux en matériaux polymère, matériaux métalliques (e.g. inox ou acier revêtu), résine, ou des cheminées verticales ou horizontales L'installation pourra comprendre un ou plusieurs réacteurs ou contenants. Dans la mesure où l'installation comprend plusieurs réacteurs ou contenants (chacun d'eux pouvant contenir une matrice réactive identique ou différente), le contrôle ponctuel ou continu des performances du système permettra de piloter un ensemble de vannes positionnées en amont et entre les silos de manière à orienter le flux de gaz vers la matrice la plus adaptée au regard de la qualité réelle (instantanée ou non) du gaz entrant.

Ainsi, des capteurs permettant de mesurer les paramètres suivants pourront être installés en amont et en aval de ce ou de ces contenants :
➢ humidité moyenne et/ou instantanée du gaz ;
➢ température moyenne et/ou instantanée du gaz ;
➢ teneurs moyennes et instantanées des différents composés gazeux d'intérêt notamment CO₂, O₂, H₂O, H₂S et les composés organiques volatils (COV) ;
➢ pression en amont et/ou en aval pour chaque contenant ;
➢ température en amont et/ou en aval pour chaque contenant (à différents niveaux) ; et/ou
➢ débit du gaz entrant dans le système.

A titre d'exemple d'installations permettant la mise en œuvre du procédé d'épuration ou de désodorisation selon la présente invention, on peut notamment citer les installations de stockage de déchets non dangereux, les stations d'épurations, les unités de méthanisation (agricole, industrielles ou d'ordures ménagère) ou bien encore les unités de gazéification.

Le procédé d'épuration ou de désodorisation de gaz selon la présente invention permet donc une épuration ou une désodorisation simple, peu coûteuse et efficace des gaz. Le procédé selon la présente invention peut ainsi être utilisé pour divers traitements industriels tels que :
➢ le prétraitement ou le traitement des biogaz pour l'élimination des composés minoritaires type H₂S, siloxanes ou les composés organiques volatils (COV) ;
> le traitement des gaz de synthèse pour l'élimination d'H₂S, de composés organiques volatils (COV), de goudrons ou de tout autre indésirable ;
> le traitement des gaz en vue de leur désodorisation par élimination notamment de composés organiques volatils (COV), d'H₂S et de NH₃ ;
➢ le traitement des fumées de combustion par élimination notamment de composés organiques volatils (COV), de métaux volatils et du CO₂ ; ou
➢ le traitement de l'air pollué par élimination notamment de composés organiques volatils (COV) ou de tout autre indésirable.

Les matrices réactives décrites précédemment peuvent donc être utilisées pour épurer ou désodoriser un gaz.

La présente invention est illustrée de façon non limitative par les exemples suivants.

### Exemple 1 : Matrice réactive

La matrice réactive utilisée à titre d'exemple est constituée de cendres de chaufferie de biomasse (CCB) récupérées sous foyer.

93 % des cendres de l'échantillon ont une granulométrie inférieure à 6 mm, et 53 % inférieure à 0,5 mm. Le pH du lixiviat est de 13,8 et sa teneur en humidité est de 33,3%. La composition massique en matière sèche de la matrice réactive est précisée dans le tableau 1 ci-dessous :

**Tableau 1**

| Composition massique (%MS) | | | | | | | | | | | |
|---|---|---|---|---|---|---|---|---|---|---|---|
| SiO₂ | Al₂O₃ | Fe₂O₃ | MnO | MgO | CaO | Na₂O | K₂O | TiO₂ | P₂O₅ | PF | Total |
| 40,65 | 4,68 | 3,07 | 0,58 | 2,79 | 25,28 | 0,63 | 6,04 | 0,46 | 1,71 | 13,45 | 99,32 |

### Exemple 2 : Procédé d'épuration ou de désodorisation de gaz selon l'invention

La matrice réactive de l'exemple 1 a été utilisée pour éliminer l'H₂S contenu dans un biogaz d'installation de stockage de déchets non-dangereux.

La concentration en H₂S au cours de la campagne d'essais était en moyenne supérieure a 200 ppmv avec des pics jusqu'à plus de 1000 ppmv. Cette variabilité est essentiellement due à des phénomènes intrinsèques à la décharge, au climat et à des actions menées pour l'exploitation du site et la gestion du biogaz.

L'installation utilisée est constituée :
- d'un débitmètre permettant de régler et mesurer le débit instantané de biogaz ;
- d'un système de barbotage thermostaté permettant de saturer le biogaz en eau à 20°C ;
- d'une colonne de 5 cm de hauteur remplie de garnissage afin d'éviter les chemins préférentiels ; et
- d'un réacteur, constitué d'une colonne en acier inoxydable contenant le matériau à étudier.

La colonne est maintenue verticalement. Le gaz à épurer est introduit par le bas de la colonne.

Un essai d'une durée de 18 jours a été réalisé avec la matrice réactive étudiée.

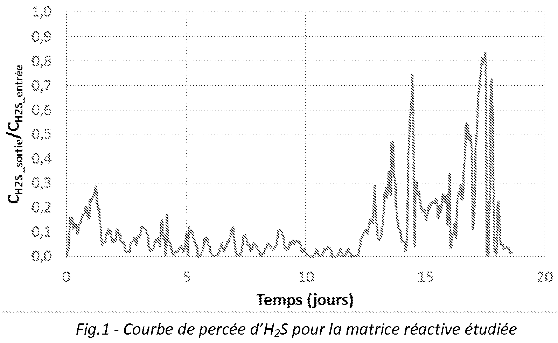

La courbe de percée, représentant l'évolution du rapport de la concentration d'H₂S en sortie sur la concentration d'H₂S en entrée en fonction du temps, est présentée figure 1.

La percée, définie ici comme le temps nécessaire pour atteindre une concentration d'H₂S en sortie de réacteur égale à 10 % de celle en entrée intervient après environ 2 h d'essai, mais le taux de rétention reste en moyenne supérieur à 90 % pendant plus de 12 jours.

Les pics de concentrations observés en sortie entre les jours 12 et 18 correspondent à des pics de concentrations d'H₂S dans le biogaz en entrée de plus de 1000 ppmv.

Lorsque la concentration d'H₂S en entrée redescend à une valeur « classique » (jour 18), le matériau recouvre un taux de rétention supérieur à 90 %.

La quantité d'H₂S retenue en fin d'essai est supérieure à 90 mg S/g, ce qui correspond à 88 % de la quantité d'H₂S passée dans le réacteur.

### Exemple 3 : Influence du taux d'humidité de la matrice

Le dispositif expérimental décrit dans l'exemple 2 a été utilisé pour évaluer l'influence du taux d'humidité de la matrice.

Trois matrices réactives dont la composition massique en matière sèche est identique à celle de l'exemple 1 mais dont on a fait varier le taux d'humidité ont été comparées :
- matrice A : taux d'humidité 8% ;
- matrice B : taux d'humidité 15% ;
- matrice C : taux d'humidité 20% ; et
- matrice D : taux d'humidité 30 %.

Les concentrations en H₂S en entrée et en sortie du réacteur ont été mesurées au cours du temps pour chaque expérience. Le taux de charge, à savoir le poids d'H₂S retenu par rapport au poids en matière sèche de la matrice réactive, a ainsi pu être déterminé pour chaque expérience.

Les résultats obtenus sont rapportés dans le tableau 2 ci-dessous.

**Tableau 2**

| | | | | |
|---|---|---|---|---|
| Taux d'humidité | 8% | 15% | 20% | 30% |
| Taux de charge | 6.7% | 9.9% | 17.8% | 25.6% |

Les résultats obtenus montrent l'influence du taux d'humidité de la matrice réactive sur sa capacité de rétention. Les matrices réactives possédant un taux d'humidité supérieur à 15% présentent des capacités épuratoires en H₂S nettement améliorées.

### Exemple 4 : Influence du taux de CaO dans la matrice

Le dispositif expérimental décrit dans l'exemple 2 a été utilisé pour évaluer l'influence du taux en poids de MS de CaO dans la matrice.

Deux matrices réactives dont la composition massique en matière sèche est identique à celle de l'exemple 1 possédant un taux d'humidité fixé à 20%, mais dont on a fait varier le taux en poids de MS de CaO, ont été comparées :
- matrice E : 23%MS de CaO ; et
- matrice F : 55%MS de CaO.

Les concentrations en H₂S en entrée et en sortie du réacteur ont été mesurées au cours du temps pour chaque expérience. Le taux de charge, à savoir le poids d'H2S retenu par rapport au poids en matière sèche de la matrice réactive, a ainsi pu être déterminé pour chaque expérience.

Les résultats obtenus sont rapportés dans le tableau 3 ci-dessous.

**Tableau 3**

| | | |
|---|---|---|
| Taux de CaO (MS) | 23% | 55% |
| Taux de charge | 18.2% | 10.4% |

Les résultats montrent que la matrice contenant 55% en poids de MS de CaO a une capacité de rétention plus faible que la matrice contenant 23% en poids de MS de CaO. La matrice contenant 23% en poids de MS de CaO présente donc de meilleures capacités épuratoires en H₂S.

## Revendications

1. Procédé d'épuration ou de désodorisation de gaz comprenant la mise en contact dudit gaz avec une matrice réactive comprenant :
- de 15% à 80% en poids de matière sèche de SiO₂; et
- de 1% à 40% en poids de matière sèche de CaO ;
le taux d'humidité de ladite matrice, c'est-à-dire le rapport du poids de l'eau, à l'exception de l'eau de cristallisation, contenue dans la matrice sur le poids total de celle-ci, variant de 20% à 80%.

2. Procédé selon la revendication 1, **caractérisé en ce que** la matrice réactive contient 15%MS à 60%MS de SiO₂.

3. Procédé selon la revendication 2, **caractérisé en ce que** la matrice réactive contient 18%MS à 40%MS de SiO₂.

4. Procédé selon l'une quelconque des revendications 1 à 3, **caractérisé en ce que** la matrice réactive contient de 10%MS à 40%MS de CaO.

5. Procédé selon la revendication 4, **caractérisé en ce que** la matrice réactive contient de 20%MS à 40%MS de CaO.

6. Procédé selon la revendication 5, **caractérisé en ce que** le taux d'humidité de la matrice réactive varie de 20% à 60%.

7. Procédé selon l'une quelconque des revendications 1 à 6, **caractérisé en ce que** le pH de la matrice réactive est supérieur ou égal à 7.

8. Procédé selon l'une quelconque des revendications 1 à 7, **caractérisé en ce que** la matrice réactive se présente sous la forme d'un mélange de poudres dont la granulométrie varie de 0,1 µm à 15 cm.

9. Procédé selon l'une quelconque des revendications 1 à 7, **caractérisé en ce que** la matrice réactive se présente sous la forme de granulés dont la taille varie de 0,1 cm à 25 cm.

10. Procédé selon l'une quelconque des revendications 1 à 9, **caractérisé en ce que** la matrice réactive comprend en outre un ou plusieurs des constituants suivants :
> jusqu'à 60%MS de Fe₂O₃ ;
➢ jusqu'à 30%MS d'Al₂O₃ ;
➢ jusqu'à 35%MS de Na₂O ;
➢ jusqu'à 50%MS de K₂O ;
➢ jusqu'à 15%MS de TiO₂ ;
➢ jusqu'à 60%MS de MgO;
➢ jusqu'à 30 %MS de SO₃ ;
➢ jusqu'à 15%MS de P₂O₅ ; et/ou
➢ jusqu'à 60%MS de C.

11. Procédé selon la revendication 10, **caractérisé en ce que** la matrice réactive comprend en outre un ou plusieurs des composés suivants :
➢ jusqu'à 10%MS d'As ;
➢ jusqu'à 10%MS de Sb ;
➢ jusqu'à 10%MS de Cd;
➢ jusqu'à 10%MS de Cr;
➢ jusqu'à 10%MS de Co ;
➢ jusqu'à 10%MS de Cu;
➢ jusqu'à 10%MS de Mn ;
➢ jusqu'à 10%MS de Ni;
➢ jusqu'à 10%MS de Pb;
➢ jusqu'à 10%MS de V ;
➢ jusqu'à 10%MS de Zn ;
➢ jusqu'à 10%MS de F ; et/ou
➢ jusqu'à 10%MS de Cl.

12. Procédé selon l'une quelconque des revendications 1 à 11, **caractérisé en ce que** le gaz à traiter possède une humidité relative avant mise en contact avec la matrice réactive supérieure ou égale à 60% et un point de rosée dans les conditions de pression du gaz est supérieur ou égal à 4°C.

## Patentansprüche

1. Verfahren zum Reinigen oder Desodorieren von Gasen, umfassend das Inkontaktbringen des Gases mit einer reaktiven Matrix, umfassend:
- 15 Gew.-% bis 80 Gew.-% Trockenmasse an SiO_{2;} und
- 1 Gew.-% bis 40 Gew.-% Trockenmasse an CaO;
wobei der Feuchtigkeitsgehalt der Matrix, d. h. das Verhältnis des Gewichts des in der Matrix enthaltenen Wassers, mit Ausnahme des Kristallisationswassers, zum Gesamtgewicht dieser zwischen 20 % und 80 % variiert.

2. Verfahren nach Anspruch 1, **dadurch gekennzeichnet, dass** die reaktive Matrix 15 %TM bis 60 %TM SiO₂ enthält.

3. Verfahren nach Anspruch 2, **dadurch gekennzeichnet, dass** die reaktive Matrix 18 %TM bis 40 %TM SiO₂ enthält.

4. Verfahren nach einem der Ansprüche 1 bis 3, **dadurch gekennzeichnet, dass** die reaktive Matrix 10 %TM bis 40 %TM CaO enthält.

5. Verfahren nach Anspruch 4, **dadurch gekennzeichnet, dass** die reaktive Matrix 20 %TM bis 40 %TM CaO enthält.

6. Verfahren nach Anspruch 5, **dadurch gekennzeichnet, dass** der Feuchtigkeitsgehalt der reaktiven Matrix zwischen 20 % und 60 % variiert.

7. Verfahren nach einem der Ansprüche 1 bis 6, **dadurch gekennzeichnet, dass** der pH-Wert der reaktiven Matrix größer oder gleich 7 ist.

8. Verfahren nach einem der Ansprüche 1 bis 7, **dadurch gekennzeichnet, dass** die reaktive Matrix in Form einer Mischung aus Pulvern vorliegt, deren Korngröße zwischen 0,1 µm und 15 cm variiert.

9. Verfahren nach einem der Ansprüche 1 bis 7, **dadurch gekennzeichnet, dass** die reaktive Matrix in Form von Granulaten vorliegt, deren Größe zwischen 0,1 cm und 25 cm variiert.

10. Verfahren nach einem der Ansprüche 1 bis 9, **dadurch gekennzeichnet, dass** die reaktive Matrix ferner einen oder mehrere der folgenden Bestandteile umfasst:
> bis zu 60 %TM Fe₂O₃;
> bis zu 30 %TM Al₂O₃;
> bis zu 35 %TM Na₂O;
> bis zu 50 %TM K₂O;
> bis zu 15 %TM TiO₂;
> bis zu 60 %TM MgO;
> bis zu 30 %TM SO₃;
> bis zu 15 %TM P₂O₅; und/oder
> bis zu 60 %TM C.

11. Verfahren nach Anspruch 10, **dadurch gekennzeichnet, dass** die reaktive Matrix ferner eine oder mehrere der folgenden Verbindungen umfasst:
> bis zu 10 %TM As;
> bis zu 10 %TM Sb;
> bis zu 10 %TM Cd;
> bis zu 10 %TM Cr;
> bis zu 10 %TM Co;
> bis zu 10 %TM Cu;
> bis zu 10 %TM Mn;
> bis zu 10 %TM Ni;
> bis zu 10 %TM Pb;
> bis zu 10 %TM V;
> bis zu 10 %TM Zn;
> bis zu 10 %TM F; und/oder
> bis zu 10 %TM Cl.

12. Verfahren nach einem der Ansprüche 1 bis 11, **dadurch gekennzeichnet, dass** das zu behandelnde Gas vor dem Inkontaktbringen mit der reaktiven Matrix eine relative Feuchtigkeit von größer gleich 60 % besitzt und ein Taupunkt unter den Druckbedingungen des Gases größer gleich 4 °C ist.

## Claims

1. A method for purifying or deodorizing gas, comprising bringing said gas into contact with a reactive matrix comprising:
- from 15% to 80% by weight of dry matter of SiO₂; and
- from 1% to 40% by weight of dry matter of CaO;
the moisture content of said matrix, that is to say the ratio of the weight of water, excluding crystallization water, contained in the matrix to the total weight thereof, ranging from 20% to 80%.

2. The method according to claim 1, **characterized in that** the reactive matrix contains from 15% DM to 60% DM of SiO₂.

3. The method according to claim 2, **characterized in that** the reactive matrix contains from 18% DM to 40% DM of SiO₂.

4. The method according to any one of claims 1 to 3, **characterized in that** the reactive matrix contains from 10% DM to 40% DM of CaO.

5. The method according to claim 4, **characterized in that** the reactive matrix contains from 20% DM to 40% DM of CaO.

6. The method according to claim 5, **characterized in that** the moisture content of the reactive matrix ranges from 20% to 60%.

7. The method according to any one of claims 1 to 6, **characterized in that** the pH of the reactive matrix is greater than or equal to 7.

8. The method according to any one of claims 1 to 7, **characterized in that** the reactive matrix is in the form of a mixture of powders having a particle size ranging from 0.1 µm to 15 cm.

9. The method according to any one of claims 1 to 7, **characterized in that** the reactive matrix is in the form of granules having a size ranging from 0.1 cm to 25 cm.

10. The method according to any one of claims 1 to 9, **characterized in that** the reactive matrix further comprises one or several of the following constituents:
➢ up to 60% DM of Fe₂O₃;
➢ up to 30% DM of Al₂O₃;
➢ up to 35% DM of Na₂O;
➢ up to 50% DM of K₂O;
➢ up to 15% DM of TiO₂;
➢ up to 60% DM of MgO;
➢ up to 30% DM of SO₃;
➢ up to 15% DM of P₂O₅; and/or
➢ up to 60% DM of C.

11. The method according to claim 10, **characterized in that** the reactive matrix further comprises one or several of the following compounds:
➢ up to 10% DM of As;
➢ up to 10% DM of Sb;
➢ up to 10% DM of Cd;
➢ up to 10% DM of Cr;
➢ up to 10% DM of Co;
➢ up to 10% DM of Cu;
➢ up to 10% DM of Mn;
➢ up to 10% DM of Ni;
➢ up to 10% DM of Pb;
➢ up to 10% DM of V;
➢ up to 10% DM of Zn;
➢ up to 10% DM of F; and/or
➢ up to 10% DM of Cl.

12. The method according to any one of claims 1 to 11, **characterized in that** the gas to be treated has a relative humidity before contact with the reactive matrix greater than or equal to 60% and a dew point, under the gas pressure conditions, greater than or equal to 4°C.
